# EUROPEAN PATENT SPECIFICATION

(11) **EP 0 524 395 B1**
(45) Date of publication and mention of the grant of the patent: **07.02.1996**
(21) Application number: 92108382.0
(22) Date of filing: 18.05.1992
(51) Int. Cl.: A61K 31/575, A61K 9/20

(54) **Sustained release compositions containing bile acids**
Gallensäuren enthaltende Arzneimittel mit verzögerter Wirkstoffabgabe
Compositions à libération contrôlée contenant des acides biliaires

(30) Priority: 22.07.1991 IT RM910550
(43) Date of publication of application: 27.01.1993
(73) Proprietor: BONISCONTRO E GAZZONE S.r.l., I-00156 Roma (IT)
(72) Inventor: Bertone, Evaristo, I-00156 Roma (IT)
(74) Representative: Minoja, Fabrizio

(56) References cited:
- EP-A- 0 293 751
- GB-A- 2 036 558

## Description

The present invention relates to sustained release pharmaceutical compositions containing ursodeoxycholic acid. The technology required for the development of sustained release pharmaceutical compositions has been well known for a long time and has been applied to many active ingredients. Sustained release compositions of bile acids and derivatives are disclosed in Italian patent no. 1,101,649 and in US patent no. 4,263,272. In the examples of said patents several kinds of compositions, which are said to be suitable to provide the desired sustained release effect, such as to allow a single daily administration, are reported.

The described compositions are mostly of non homogeneous kind (two-layer sugar pills, capsules containing two or three small tablets having different release characteristics, multilayer tablets or tablets with concentric cores) which are difficult and expensive to prepare.

For other type of active ingredients the use of sodium and calcium alginate mixtures has been suggested, said compounds form gels which are particularly thick when in contact with gastrointestinal juices (GB 1.355.985 - U.S. 3.640.741 - JP 76415 and 76413). K.A. Khan et al., J. Pharm. Sci. 64, 166 (1975) E. Shotton et al., J. Pharm. Sci. 65, 1170 (1976) A.E. Abotaleb et al., Pharmazie 38, 473 (1983). Recently, in EP 0188040 a sodium and calcium alginate, with a well defined composition, in admixture with sodium alginate, was proposed.

Italian patent no. 1,211,645 discloses the use of agar as gelling agent in the preparation of solid sustained release pharmaceutical compositions. Said patent discloses tabletted matrixes obtained by using the properties of agar and gels therefrom (i.e. insolubility in cold water, very slow swelling and melting, softening only at 70°-80°C, good physical resistance, permeability independent from pH, respectively). The so obtained matrixes, when in contact with aqueous media, swell thus forming very viscous gels; therefore the medicaments contained in the tablet are released in a continuous mode by diffusing through the so formed gel.

Generally, in the preparation of the matrixes, several gums, among which tracaganth gum, karaya gum, alginates, arabic gum and also powdery methacrylic polymers (Eudragit PM) are combined with agar, and in many cases advantages are achieved by filming with rapidly disgregating coatings, enteric coatings, sustained release coating independent from pH, using cellulose derivatives or methacrylic polymers or other equivalents.

The solution of the problem proposed in IT 1,211,645, even if perfect from several point of views, fails when applied as such to bile acids, which are practically water-insoluble.

It has now been found that the addition of variable amounts of basic buffers to the agar used as gelling agent according to IT 1,211,645, allows to control the diffusion of the active ingredient through the gel, therefore gradually releasing it in the etching liquids.

The buffers to be used consist of alkali metal salts (for example sodium phosphate, citrate, tartrate), amine salts (such as triethanolamine, methylglucamine, tromethamine, diethylendiamine, ethylendiamine phosphate or citrate, etc.); in some cases the addition of appropriate amounts of free bases to neutral salts could be convenient, however, the final pH of the product treated with water must be lower than 8.

Therefore, the compositions according to the invention have the following composition:
a) Active ingredient: a bile acid, particularly ursodeoxycholic acid, in a dosage ranging preferably from 200 to 600 mg;
b) Agar in percentage amounts from 3% to 10% of the active ingredient;
c) One or more substances suitable for gel formation, such as tracaganth gum, karaya gum, alginates, arabic gum, methacrylic polymers;
d) A basic buffer in molar ratio with the active ingredient ranging from 0.05:1 to 1:1;
e) Optional binding agents, diluents, lubricants in amounts normally used according to the well-known preparative technique;
f) Optional fast-disgregating, or gastroresistant or slow release coatings.

The amount of substances c) is normally from 1 to 20 times the amount of the used agar.

The sum of the amounts of gelling agents, agar included, must be between 10 and 65% of the active ingredient.

The preparation of the compositions of the invention requires no particular equipments and provides the following steps:
- wet granulation
- drying
- dry granulation
- sifting and tabletting
- optional filming.

The obtained tablets, due to different reasons, such as protection from moisture, fluidity, unpleasant flavour, etc, can be filmed with fast disgregating films, elastic and permeable coatings to protect the gel mechanically.

Methacrylic polymers are the most advantageous materials, however, cellulose derivatives and other equivalent materials can also be used.

Gastroresistant coatings are particularly suitable for tablets containing a buffer, in order to avoid that the high acid content of gastric juice alters the expected buffering effect.

The present invention allows to achieve the following advantages:
1) The daily dosage is administered by means of a single rather small tablet.
2) Simple manufacturing consisting in the tabletting of just one type of granulate, said operation may be performed with a normal equipment available in every shop.
3) The package consists of a single type of tablet directly blistered or in container.

### Examples

The following examples are intended to illustrate the present invention, without limiting it in any way.

### Example 1

| | |
|---|---|
| Ursodeoxycholic acid | 450.00 mg |
| Tracaganth gum | 150.00 mg |
| Agar | 30.00 mg |
| Sodium citrate (anhydrous) | 30.00 mg |
| Citric acid | 5.00 mg |
| Magnesium stearate | 10.00 mg |
| Methacrylic copolymers | 25.00 mg |
| Titanium dioxide | 1.00 mg |
| Dibutyl phtalate | 0.50 mg |
| E 172 | 0.10 mg |

The method for the preparation was as above disclosed, followed by an acid resistant coating.

The tablets were tested in vitro according to USP XXI method.
- 1^{st} h at pH 1.5 - no detectable variation
- 2^{nd} h at pH 6.8 - 9% release
- 3^{rd} h at pH 6.8 - 11.5% release
- 4^{th} h at pH 6.8 - 10.5% release
- 5^{th} h at pH 6.8 - 12% release
- 6^{th} h at pH 6.8 - 12% release
- 7^{th} h at pH 6.8 - 10.5% release
- 8^{th} h at pH 6.8 - 12.5% release
- 9^{th} h at pH 6.8 - 11% release
- 10^{th} h at pH 6.8 - 9% release

### Example 2

| | |
|---|---|
| Ursodeoxycholic acid | 225.00 mg |
| Tracaganth gum | 80.00 mg |
| Agar | 10.00 mg |
| Sodium phosphate (anhydrous) | 23.00 mg |
| Magnesium stearate | 5.00 mg |
| Methacrylic copolymers | 12.50 mg |
| Titanium dioxide | 0.50 mg |
| Dibutyl phtalate | 0.25 mg |
| E 172 | 0.05 mg |

The release test according to USP XXI gave results similar to Example 1, characterized by a very regular release.

Preliminary bioavalability tests, carried out on human beings comparing sustained release tablets with normal tablets, single administered and multiadministered, respectively, gave results which were in good agreement with the ones from in vitro release test.

## Claims

1. Sustained release pharmaceutical compositions containing bile acids as active ingredient, said composition containing:
a) agar in percentage amounts from 3% to 10% of the active ingredient;
b) one or more substances suitable for gel formation, such as tracaganth gum, karaya gum, alginates, arabic gum, methacrylic polymers;
c) a basic buffer in molar ratio with respect to the active ingredient ranging from 0.05:1 to 1:1, the final pH of the product treated with water being lower than 8;
d) optional binding agents, diluents, lubricants;
e) optional fast disgregating, or gastroresistant or slow release coatings.

2. Compositions according to claim 1 wherein ursodeoxycholic acid is the bile acid.

3. Compositions according to claim 1 or 2, wherein the active ingredient is contained in amounts ranging from 200 to 600 mg.

4. Compositions according to anyone of the preceding claims wherein tracaganth gum is the substance suitable for the gel formation.

## Patentansprüche

1. Pharmazeutische Zusammensetzungen mit anhaltender Freisetzung (Depot-Präparate), die als aktiven Bestandteil (Wirkstoff) Gallensäuren enthalten, wobei die Zusammensetzung enthält:
a) Agar in Mengen von 3 bis 10 %, bezogen auf den aktiven Bestandteil (Wirkstoff);
b) eine oder mehr Substanzen, die für die Gelbildung geeignet sind, wie Traganth-Gummi, Karaya-Gummi, Alginate, Gummiarabicum, Methacrylpolymere;
c) einen basischen Puffer in einem Molverhältnis, bezogen auf den aktiven Bestandteil (Wirkstoff), in dem Bereich von 0,05:1 bis 1:1, wobei der End-pH-Wert des mit Wasser behandelten Produkts unter 8 liegt;
d) gegebenenfalls Bindemittel, Verdünnungsmittel, Schmiermittel (Gleitmittel); und
e) gegebenenfalls schnell zerfallende Überzüge oder im Magen beständige Überzüge oder Überzüge mit einer langsamen Freisetzung.

2. Zusammensetzungen nach Anspruch 1, in denen die Gallensäure Ursodeoxycholsäure ist.

3. Zusammensetzungen nach Anspruch 1 oder 2, in denen der aktive Bestandteil (Wirkstoff) in Mengen in dem Bereich von 200 bis 600 mg enthalten ist.

4. Zusammensetzungen nach einem der vorhergehdnden Ansprüche, in denen Traganth-Gummi die für die Gelbildung geeignete Substanz ist.

## Revendications

1. Compositions pharmaceutiques à libération entretenue contenant des acides biliaires à titre de principe actif, lesdites compositions contenant :
a) une gélose dans des quantités en pourcentage de 3% à 10% du principe actif ;
b) une ou plusieurs substances aptes à former un gel, telles que la gomme adragante, la gomme de sterculia, les alginates, la gomme arabique, les polymères méthacryliques ;
c) un tampon basique dans un rapport molaire par rapport au principe actif, de 0,05:1 à 1:1, le pH final du produit traité avec de l'eau étant inférieur à 8 ;
d) éventuellement, des agents de liaison, des diluants, des lubrifiants ;
e) éventuellement, des enrobages à désagrégation rapide ou gastrorésistants ou à libération lente.

2. Compositions selon la revendication 1, dans lesquelles l'acide ursodésoxycholique est l'acide biliaire.

3. Compositions selon la revendication 1 ou 2, dans lesquelles le principe actif est présent dans des quantités de 200 à 600 mg.

4. Compositions selon l'une quelconque des revendications précédentes, dans lesquelles la gomme adragante est la substance apte à former le gel.
